# EUROPEAN PATENT APPLICATION

(11) **EP 4 011 375 A1**
(43) Date of publication of application: **15.06.2022**
(21) Application number: 20850324.3
(22) Date of filing: 05.08.2020
(51) Int. Cl.: A61K 31/47, A61K 47/20, A61K 47/12, A61K 9/20, A61K 9/36, A61P 35/00

(54) **PHARMACEUTICAL COMPOSITION CONTAINING NITROXOLINE, NITROXOLINE ORAL SOLID TABLET, PREPARATION METHOD THEREFOR AND USE THEREOF**

(30) Priority: 05.08.2019 CN 201910716938
(71) Applicant: Jiangsu Yahong Meditech Co., Ltd., Taizhou, Jiangsu 225316 (CN); Shanghai Yahong Meditech Co., Ltd., Shanghai 201203 (CN)
(72) Inventor: LIU, Jianghua, Taizhou, Jiangsu 225316 (CN); GUO, Yushen, Shanghai 201203 (CN); SHEN, Shuai, Taizhou, Jiangsu 225316 (CN); ZHU, Wei, Taizhou, Jiangsu 225316 (CN)
(74) Representative: EP&C
(86) International application number: PCT/CN2020/107167
(87) International publication number: WO 2021/023231

(57) **Abstract**

A pharmaceutical composition containing nitroxoline for the treatment of bladder cancer, a nitroxoline oral solid tablet, a preparation method therefor and use thereof. The pharmaceutical composition comprises nitroxoline, a filler, a disintegrating agent, a binder, and a lubricant. The lubricant is selected from one or two of sodium dodecyl sulfate and sodium stearyl fumarate. The pharmaceutical composition has a moderate dissolution rate, can avoid the burst release phenomenon, is moisture-proof and impermeable, has good stability, is secure and effective, is convenient to take, has strong patient compliance, and meets the requirements of being an oral solid tablet. The preparation method has stable production process, good reproducibility, easy mass production, and good clinical use value and social benefits.

## Description

### FIELD OF THE INVENTION

The present invention relates to a pharmaceutical composition containing nitroxoline for the treatment of bladder cancer, a nitroxoline oral solid tablet, a preparation method therefor and use thereof.

### BACKGROUND OF THE INVENTION

Bladder cancer is one of the malignant tumors that seriously threaten human health, and is the most common malignant tumor of the urinary system.

According to the data published in "Chinese guidelines for diagnosis and treatment of urothelial carcinoma of bladder 2018", the global incidence rate of bladder cancer is the 11th among all malignant tumors, wherein the incidence rate is 9.0/100,000 for males which is the 7th among malignant tumors in males; and 2.2/100,000 for females which is lower than 10th among malignant tumors in females; the mortality rate is the 13th among all tumors, 3.2/100,000 for males and 0.9/100,000 for females respectively, and the mortality rate is the 9th among malignant tumors in males.

According to statistics from the National Cancer Registry, the incidence rate of bladder cancer in 2009 is 6.61/100,000, and the population-standardized rate is 3.03/100,000. The incidence rates for males and females are 11.41/100,000 and 3.51/100,000 respectively, and that for males is 3.3 times that for females. The mortality rate is 2.60/100,000, 3.75/100,000 for males and 1.24/100,000 for females respectively, and the ratio of male to female is 2.97:1.

In 2016, it is expected to have 80,500 new cases of bladder cancer in China, 62,100 for males (which is the 6th in malignant tumors in males) and 18,400 for females; and there will be 32,900 deaths, including 25,100 males (which is the 11th in malignant tumor in males) and 7,800 females. Currently, there are approximately 2.7 million surviving bladder cancer patients worldwide. The incidence of bladder cancer in China is the 8th among malignant tumors, wherein, the incidence for males is higher, ranking the 6th in malignant tumors.

Bladder cancer can be classified into non-muscular invasive bladder cancer and muscular invasive bladder cancer. Non-muscular invasive bladder cancer (previously known as superficial bladder cancer) accounts for 75%-85%, and muscular invasive bladder cancer accounts for 15%-25%. Currently, the major therapy for non-muscular invasive bladder cancer is bladder tumor resection and postoperative chemotherapy infusion. However 10%-67% of patients will relapse within 12 months, and 24%-84% of patients will relapse within 5 years.

The currently marketed drugs for treating bladder cancer are basically bladder perfusion drugs. During treatment, the drug must be infused into the patient's bladder through a urethral cannula under the operation of a professional nurse. This method of administration requires patients (and families) to go to the hospital frequently, and will cause much pain to patients, especially male patients. Therefore, according to the current clinical and market demand, it is urgent to develop a drug delivery formulation with strong patient compliance, for example oral solid tablets.

Nitroxoline, the chemical name of which is 5-nitro-8-hydroxyquinoline, was developed as an oral antibiotic drug in the 1960s. It was mainly used for urinary system infections and had a relatively safe history of use before being replaced due to discovery and use of new antibiotics. In recent years, new studies have found that nitroxoline can simultaneously inhibit the methionine aminopeptidase MetAP2 and the silence information regulator 2-related enzyme SIRT1 in vascular endothelial cells, exerting a synergistic inhibitory effect on tumor angiogenesis, as well as an inhibitory effect on the proliferation of tumor cells. Therefore, nitroxoline has been re-developed to treat tumors including bladder cancer.

However, for oral solid tablets, burst release of the drug may increase the incidence of side effects, while slow release may lead to incomplete absorption and low bioavailability. Therefore, it is necessary to develop a nitroxoline oral solid tablet with a moderate release rate.

In addition, the nitroxoline compound has relatively strong mobility and permeability. The traditional method uses sugar coating technology which will increase the weight of the tablet core by 50%-100% to prevent the migration and penetration of the drug. However, the sugar coating process is a relatively old preparation process with complicated process steps, which is time-consuming and labor-intensive, requires much experience, and has difficulties in quality control and industrial mass production. In addition, as the sugar coating contains a large amount of powdered sugar and talc, it is not suitable for long-term administration in middle-aged, elderly and diabetic patients. Therefore, it is necessary to develop a nitroxoline oral solid tablet suitable for mass production and suitable for use in middle-aged, elderly and diabetic patients.

### SUMMARY OF THE INVENTION

In order to solve the technical problem that there is no oral solid tablet containing nitroxoline with a moderate release rate in the prior art, the present invention provides a pharmaceutical composition containing nitroxoline, a nitroxoline oral solid tablet, a preparation method therefor and use thereof. The nitroxoline oral solid tablet of the present invention has a moderate dissolution rate (10 min dissolution≤65%, 60 min dissolution≥80%) which can avoid the burst release phenomenon, and is moisture-proof and impermeable. It also has the properties of good stability, safe and effective, convenient to take, and strong patient compliance. These will meet the requirements of being an oral solid tablet. The preparation method of the present invention has the properties of stable production process, good reproducibility, and easy mass production, which will provide good clinical use value and social benefits. In addition, the nitroxoline tablet of the present invention is quickly absorbed after oral administration (Tₘₐₓ=1.5 to 2 hours), and has a high degree of absorption (about 90%).

Thus, the present invention provides a pharmaceutical composition containing nitroxoline, which comprises nitroxoline and a lubricant; the lubricant is selected from one or two of sodium dodecyl sulfate and sodium stearyl fumarate.

The present invention further provides a pharmaceutical composition containing nitroxoline, which comprises nitroxoline, a filler, a disintegrating agent, a binder and a lubricant; the lubricant is selected from one or two of sodium dodecyl sulfate and sodium stearyl fumarate.

In the above pharmaceutical composition, the mass percentage of the nitroxoline in the pharmaceutical composition is preferably 30%-65%, for example 35%, 40%, 45%, 50%, 55% or 60%, more preferably 40%-60%.

In the above pharmaceutical composition, the filler is preferably one or more of lactose, starch, pregelatinized starch, microcrystalline cellulose, dextrin, mannitol, calcium hydrophosphate, mannitol and sorbitol; more preferably one or more of lactose, starch and microcrystalline cellulose; further more preferably lactose and/or starch; still more preferably lactose and starch; and the mass percentage of lactose in the pharmaceutical composition is 10%-25%, for example 17.5%, and the mass percentage of starch in the pharmaceutical composition is 23%-30%, for example 26.5%. The lactose is preferably lactose monohydrate. The starch is preferably corn starch.

In the above pharmaceutical composition, the mass percentage of the filler in the pharmaceutical composition is preferably 25%-65%, for example 30%, 33%, 40%, 42.5%, 43.5%, 44%, 44.5%, 45%, 50%, 52%, 55% or 60%, more preferably 33%-55%.

In the above pharmaceutical composition, the lactose is preferably lactose monohydrate. The starch is preferably corn starch.

In the above pharmaceutical composition, the disintegrating agent is preferably one or more of hydroxypropyl cellulose, cross-linked polyvinylpyrrolidone, sodium carboxymethyl starch and sodium croscarmellose, more preferably hydroxypropyl cellulose, further more preferably low-substituted hydroxypropyl cellulose.

In the above pharmaceutical composition, the mass percentage of the disintegrating agent in the pharmaceutical composition is preferably 1%-5%, for example 2%, 2.5%, 3% or 4%, more preferably 2%-4%, further more preferably 2%-3%.

In the above pharmaceutical composition, the binder is preferably one or more of starch, povidone, hydroxypropyl methylcellulose, hydroxypropyl cellulose and sodium carboxymethyl cellulose, more preferably starch and/or hydroxypropyl methylcellulose, further more preferably starch. The starch is preferably corn starch. The hydroxypropyl methylcellulose is preferably E3 hydroxypropyl methylcellulose.

In the above pharmaceutical composition, the mass percentage of the binder in the pharmaceutical composition is preferably 1%-4%, for example 1.5%, 2%, 3% or 3.5%, more preferably 1%-3%.

In the above pharmaceutical composition, the lubricant is preferably sodium dodecyl sulfate. It is surprisingly found that when sodium dodecyl sulfate is used as a lubricant, it simultaneously plays a dual role of lubrication and solubilization, which can successfully solve the technical difficulty that the resulting pharmaceutical composition ages and does not disintegrate under high temperature conditions in the influencing factor test. The obtained pharmaceutical composition has properties of good dissolution and stability and the like.

In the above pharmaceutical composition, the mass percentage of the lubricant in the pharmaceutical composition is preferably 0.5%-4%, for example 1%, 2%, 3% or 4%, more preferably 1%-2%.

In the above pharmaceutical composition, the pharmaceutical composition preferably comprises a coating agent, which can be selected from one or more of Opadry, polyvinyl alcohol, Eudragit and gastric-soluble film coating premix, preferably gastric-soluble film coating premix.

The gastric-soluble film coating premix generally comprises titanium dioxide, talc, polyethylene glycol, hydroxypropyl methylcellulose and lake. For example, the gastric-soluble film coating premix is a mixture consisting of titanium dioxide, talc, polyethylene glycol 6000, hydroxypropyl methylcellulose and tartrazine aluminum lake.

The weight gain of the coating agent is preferably 7%-15%, more preferably 10%-12%, for example 11%.

In the above pharmaceutical composition, the pharmaceutical composition preferably comprises the following components in the following mass percentage: 30%-65% nitroxoline, 25%-65% filler, 1%-5% disintegrating agent, 1%-4% binder and 0.5%-4% lubricant. More preferably, the pharmaceutical composition comprises the following components in the following mass percentage: 40%-60% nitroxoline, 33%-55% filler, 2%-3% preferably 2%-2.5% disintegrating agent, 1%-3% binder and 1%-2% lubricant. Further more preferably, in the pharmaceutical composition, the filler is lactose and starch, and the mass percentage of lactose in the pharmaceutical composition is 10%-25%, the mass percentage of starch in the pharmaceutical composition is 23%-30%; the disintegrating agent is hydroxypropyl cellulose, the binder is starch, and the lubricant is sodium dodecyl sulfate. Still more preferably, the pharmaceutical composition further comprises a gastric-soluble film coating premix as described above as a coating with a weight gain percentage of 10%-12%, preferably 11%. In the above technical solution, the lactose is preferably lactose monohydrate. The starch is preferably corn starch. The hydroxypropyl cellulose is preferably low-substituted hydroxypropyl cellulose. The hydroxypropyl methylcellulose is preferably E3 hydroxypropyl methylcellulose. In the above technical solution, the obtained pharmaceutical composition has not only a moderate dissolution rate (10 min dissolution≤65%, 60 min dissolution≥80%), but also good stability.

The present invention further provides a nitroxoline oral solid tablet, which comprises nitroxoline as an active ingredient, a filler, a disintegrating agent, a binder and a lubricant; wherein the lubricant is selected from one or two of sodium dodecyl sulfate and sodium stearyl fumarate.

In some preferred embodiments, the tablet comprises 30-60% preferably 45-55% nitroxoline, 25-65% preferably 30-55% filler, 1-5% preferably 2-4% disintegrating agent, 1-4% preferably 1.5-3% binder and 0.5-4% preferably 1-3% lubricant by mass based on the total weight of the tablet.

The present invention also provides a nitroxoline oral solid tablet, which comprises nitroxoline as an active ingredient, a filler, a disintegrating agent, a binder and a lubricant, the tablet is further coated with one or more layers of coating agent; wherein the lubricant is selected from one or two of sodium dodecyl sulfate and sodium stearyl fumarate.

In some preferred embodiments, the tablet comprises 30-65% preferably 45-55% nitroxoline, 25-65% preferably 30-55% filler, 1-5% preferably 2-4% disintegrating agent, 1-4% preferably 1.5-3% binder and 0.5-4% preferably 1-3% lubricant by mass based on the total weight of the tablet; and the percentage of weight gain of the coating agent is 7-15%, preferably 9-12% by weight.

The weight gain percentage herein refers to the difference between the average tablet weight after coating and before coating as a percentage of the average tablet weight before coating. The equation for calculation is: weight gain percentage = (average tablet weight after coating - average tablet weight before coating) / average tablet weight before coating × 100%.

In some preferred embodiments, the filler is selected from one or more of lactose, starch, pregelatinized starch, microcrystalline cellulose, dextrin, mannitol, calcium hydrophosphate, mannitol and sorbitol, preferably lactose and/or starch. The lactose is preferably lactose monohydrate. The starch is preferably corn starch.

In some preferred embodiments, the filler is lactose and starch, wherein the mass percentage of lactose is 10%-30%, preferably 15-25%, and the mass percentage of starch is 15-35%, preferably 20-30% by mass based on the total weight of the tablet.

In some preferred embodiments, the disintegrating agent is selected from one or more of hydroxypropyl cellulose, cross-linked polyvinylpyrrolidone, sodium carboxymethyl starch and sodium croscarmellose, preferably hydroxypropyl cellulose, more preferably low-substituted hydroxypropyl cellulose.

In some preferred embodiments, the binder is selected from one or more of starch, povidone, hydroxypropyl methylcellulose, hydroxypropyl cellulose and sodium carboxymethyl cellulose, preferably starch and/or hydroxypropyl methylcellulose. The starch is preferably corn starch. The hydroxypropyl methylcellulose is preferably E3 hydroxypropyl methylcellulose.

When the above nitroxoline oral solid tablet comprises a coating agent, the coating agent can be selected from one or more of Opadry, polyvinyl alcohol, Eudragit and gastric-soluble film coating premix, preferably gastric-soluble film coating premix.

"Opadry" is an original, customizable, one-step powder mixing full-formulation film coating system of Colorcon, which comprises the required polymers, plasticizers and coloring agents.

"Eudragit" is the trade name of synthetic pharmaceutical excipient, which includes methacrylic acid copolymers and methacrylate copolymers. It is widely used in gastric-soluble coating of pharmaceutical preparations.

The gastric-soluble film coating premix refers to a mixture made of a variety of pharmaceutical excipients, the coating film made from which can be quickly dissolved in gastric juice. It can be used as a coating material for solid preparation of Chinese and Western medicines, and plays the role of taste masking, moisture-proofing and anti-cracking. The gastric-soluble film coating premix generally comprises titanium dioxide, talc, polyethylene glycol, hydroxypropyl methylcellulose and lake. For example, the gastric-soluble film coating premix is a mixture consisting of titanium dioxide, talc, polyethylene glycol 6000, hydroxypropyl methylcellulose and tartrazine aluminum lake.

In some preferred embodiments, the tablet comprises 30-65% nitroxoline as active ingredient, 10-30% lactose and/or 15-35% starch as filler, 1-5% hydroxypropyl cellulose as disintegrating agent, 1-4% starch /or hydroxypropyl methylcellulose as binder, and 0.5-4% sodium dodecyl sulfate and/or sodium stearyl fumarate as lubricant by mass based on the total weight of the tablet. Preferably, the tablet comprises 45-55% nitroxoline as active ingredient, 15-25% lactose and/or 20-30% starch as filler, 2-4% hydroxypropyl cellulose as disintegrating agent, 1.5-3% starch and/or hydroxypropyl methylcellulose as binder, and 1-3% sodium dodecyl sulfate and/or sodium stearyl fumarate as lubricant by mass based on the total weight of the tablet, and the tablet is further coated with gastric-soluble film coating premix, wherein the percentage of weight gain is 7-15%, preferably 9-12% by weight.

The content of the binder herein refers to the content of the solid binder in the tablet, and when preparing the nitroxoline oral tablet, the binder can be in the form of a solution.

The present invention further provides a preparation method of the pharmaceutical composition according to the present invention, which comprises the following steps:
S1. the nitroxoline, filler and disintegrating agent are mixed, which is then mixed with the solution containing the binder; the mixture is subject to wet granulation, drying and granulation to give the granules;
S2. the granulated granules are mixed with the lubricant and then subjected to tableting to give the tablets.

When the pharmaceutical composition contains a coating agent, coating may be performed after the tableting of step S2.

The present invention further provides a preparation method of the nitroxoline oral solid tablet according to the present invention, which comprises the following steps:
Step 1) the nitroxoline, filler, disintegrating agent, binder and lubricant are weighted;
Step 2) the nitroxoline bulk drug, diluent, disintegrating agent, binder and lubricant are sieved;
Step 3) an aqueous solution of the binder is prepared with purified water;
Step 4) the nitroxoline is mixed with the filler and disintegrating agent, then the aqueous solution of the binder prepared in step 3) is added; the mixture is subjected to wet granulation, drying and granulation to prepare uniform granules;
Step 5) the lubricant is added to the granules prepared in step 4) and mixed; the mixture is subjected to tableting to give the nitroxoline oral solid tablets.

The present invention further provides a preparation method of the nitroxoline oral solid tablet according to the present invention, which comprises the following steps:
Step 1) the nitroxoline, filler, disintegrating agent, binder, lubricant and coating agent are weighted;
Step 2) the nitroxoline, diluent, disintegrating agent, binder and lubricant are sieved;
Step 3) an aqueous solution of the binder is prepared with purified water;
Step 4) the nitroxoline is mixed with the filler and disintegrating agent, then the aqueous solution of the binder prepared in step 3) is added; the mixture is subjected to wet granulation, drying and granulation to prepare uniform granules;
Step 5) the lubricant is added to the granules prepared in step 4) and mixed; the mixture is subjected to tableting to give the nitroxoline oral solid tablet cores;
Step 6) the nitroxoline oral solid tablet core prepared in step 5) is coated with a coating agent to prepare the nitroxoline oral solid tablets.

In the above preparation method, in step 3), the binder is preferably prepared into an aqueous solution with a mass concentration of 5-10% with purified water.

In the above preparation method, when coating, the coating agent is preferably prepared into an aqueous solution with a mass concentration of 12-16% with purified water; coating of one or more layers is carried out in a coating pan at a speed of 3-15 rpm, an air inlet temperature of 55-75°C, a bed temperature at 30-50°C and a atomization pressure at 0.2-0.3 MPa.

The present invention further provides use of the nitroxoline oral solid tablet according to the present invention in the preparation of medicaments for the treatment of bladder cancer.

The "tablet core" herein refers to the nitroxoline oral solid tablet that has not undergone a coating step, for example, the uncoated tablet prepared in step 1) to step 5) as described above. In addition, the "tablet core" herein can also refer to the nitroxoline oral solid tablet itself.

The "nitroxoline oral solid coated tablet", "nitroxoline oral tablet", "nitroxoline solid tablet" and "nitroxoline tablet" herein can all be expressed as "nitroxoline oral solid tablet".

The positive and progressive effects of the present invention are that the pharmaceutical composition and the nitroxoline oral solid tablet of the present invention have a moderate dissolution rate (10 min dissolution≤65%, 60 min dissolution ≥80%) which can avoid the burst release phenomenon, and they are moisture-proof and impermeable, and have the properties of good stability, safe and effective, convenient to take, and strong patient compliance, which will meet the requirements of being an oral solid tablet. The preparation method of the present invention has the properties of stable production process, good reproducibility, and easy mass production, which will provide good clinical use value and social benefits.

### DESCRIPTION OF THE DRAWINGS

Figure 1 is the dissolution profile of the nitroxoline oral solid tablet core prepared in Comparative Example 1 in 0.1 mol/L hydrochloric acid.
Figure 2 is the dissolution profile of the nitroxoline oral solid tablet core prepared in Example 1 in 0.1 mol/L hydrochloric acid.
Figure 3 is the dissolution profile of the nitroxoline oral solid tablet of the present invention prepared in Example 6 in 0.1 mol/L hydrochloric acid.

### DETAILED DESCRIPTION OF THE INVENTION

The embodiments of the present invention will be described in detail below by the examples. However those skilled in the art should understand that the following examples are only used to illustrate the present invention and should not be regarded as limiting the scope of the present invention. If no specific conditions are indicated in the examples, it is carried out in accordance with the conventional conditions or the conditions recommended by the manufacturer. Similar commercially available products with the same functions can also be used as the raw materials or instruments besides those with specified manufacturer.

### Experimental materials:

| Name | Type/grade | Source |
|---|---|---|
| Water | Purified water | Shengjieda, Taizhou |
| Hydrochloric acid | AR | Sinopharm Chemical Reagent Co., Ltd. |
| Nitroxoline | Bulk drug | Self-manufactured by Asieris Pharmaceuticals |
| Lactose | Monohydrate, 312 | FOREMOST FARMS USA |
| Starch | Corn starch | Liaoning Dongyuan Pharmaceuticals |
| Hydroxypropyl cellulose | Low-substituted | Huzhou Zhanwang Pharmaceuticals |
| Sodium dodecyl sulfate | Pharmaceutical excipient | Hunan Jiudian Pharmaceutical |
| Microcrystalline cellulose | PH101 | Huzhou Zhanwang Pharmaceuticals |
| Cross-linked PVP | K30 | BASF, Germany |
| Pregelatinized starch | Pharmaceutical excipient | Huzhou Zhanwang Pharmaceuticals |
| Sodium carboxymethyl starch | Pharmaceutical excipient | Huzhou Zhanwang Pharmaceuticals |
| Cross-linked sodium carboxymethyl starch | Pharmaceutical excipient | Anhui Sunhere |
| Magnesium stearate | Pharmaceutical excipient | Huzhou Zhanwang Pharmaceuticals |
| Stearic acid | Pharmaceutical excipient | Huzhou Zhanwang Pharmaceuticals |
| Sodium stearyl fumarate | S96 | Taiwan Standard |
| Hydroxypropyl methylcellulose | E3 | Dow, USA |
| Gastric-soluble film coating premix | Yingyimei | Beijing Yingmao Pharmaceuticals |
| Opadry | Opadry | Colorcon |
| Eudragit | L100 | Evonik Specialty Chemicals |

The gastric-soluble film coating premix is a mixture consisting of titanium dioxide, talc, polyethylene glycol 6000, hydroxypropyl methylcellulose and tartrazine aluminum lake.

### Experimental instruments:

| Name | Model | Source |
|---|---|---|
| Wet granulation pan | G10 | Shenzhen Xinyite Technology Co., Ltd. |
| Oscillating granulator | YK-6 | Jiangyin Dry Equipment Manufacture Co., Ltd. |
| Blast drying oven | DHG-9240A | Shanghai Yiheng Scientific Instrument Co., Ltd. |
| Rotary tablet press | ZP-9 | Taizhou Tiantai Pharmacy Machines |
| UV spectrophotometer | UV-2700 | Shimadzu, Japan |
| Coating pan | BY-300 | Taizhou Jintai Pharmaceutical Machinery Co., Ltd. |
| Liquid chromatograph | LC-20A | Shimadzu, Japan |
| Centrifuge | LC-04C | Zenith Lab (Jiangsu) Co., Ltd. |

### Comparative Example 1 Preparation of nitroxoline oral solid tablet cores

In order to obtain a formula for tablet core that has a moderate dissolution rate and avoids burst release, the types and proportions of excipients are selected. Based on the total weight of the tablet core, the weight percentages of various components are shown in Table 1 below.

**Table 1 Weight percentage of various components in each formula**

| Component/Formula | 1 | 2 | 3 | 4 | 5 | 6 | 7 |
|---|---|---|---|---|---|---|---|
| Nitroxoline (%) | 50 | 50 | 50 | 50 | 50 | 50 | 50 |
| Lactose (%) | 17.5 | 17.5 | 16 | 16 | 16 | 16 | 17.5 |
| Starch (%) | - | - | 30 | 28.5 | 28.5 | 28.5 | 28 |
| Microcrystalline cellulose (%) | 15 | - | - | - | - | - | - |
| Pregelatinized starch (%) | 15 | 15 | - | - | - | - | - |
| Starch (binder) (%) | 1.5 | 1.5 | 3 | 3 | 3 | 3 | 1.5 |
| Hydroxypropyl cellulose (%) | - | 15 | - | - | - | - | 2 |
| Cross-linked PVP (%) | - | - | - | 1.5 | - | - | - |
| Sodium carboxymethyl starch (%) | - | - | - | - | 1.5 | - | - |
| Cross-linked sodium carboxymethyl starch (%) | - | - | - | - | - | 1.5 | - |
| Magnesium stearate (%) | 1 | 1 | 1 | 1 | 1 | 1 | 1 |

Preparation method of the nitroxoline oral solid tablet core:
(1) The nitroxoline, filler, disintegrating agent, binder and lubricant were respectively weighed according to the amount in the table above;
(2) The nitroxoline was sieved through a 10 mesh sieve and the other components were sieved through a 60 mesh sieve;
(3) Each amount of the binder was prepared into an aqueous solution at 5% mass concentration with purified water;
(4) Each amount of nitroxoline, filler and disintegrating agent were put into a wet granulation pan and mixed well, and then the aqueous solution of the binder prepared in step (3) was added. The mixture was stirred at high speed and discharged, which was then subjected to wet granulation by an oscillating granulator equipped with 20 mesh sieve. The granules were dried in a 60°C blast drying oven and then subjected to dry granulation by an oscillating granulator equipped with 20 mesh sieve to give an uniform granule;
(5) Each amount of the lubricant was added to the granules prepared in step (4) and mixed. The mixture was tableted by a rotary tablet press (equipped with 6 mm round punch) to give the nitroxoline oral solid tablet core.

### Comparative Example 2 Dissolution test of the nitroxoline oral solid tablet cores prepared in Comparative Example 1

According to the Chinese Pharmacopoeia 2015 edition, Volume IV, General Requirements 0931, 1000 mL of 0.1 mol/L HCl was used as the dissolution medium, and the rotating speed of the automatic dissolution tester (model RC8MD, TDTF Company) was 50 revolutions per minute. The samples were collected at 5, 10, 20, 30, 45 and 60 minutes respectively, and the absorbance was measured at a wavelength of 369 nm by an UV spectrophotometer. The dissolution was calculated according to the external standard method.

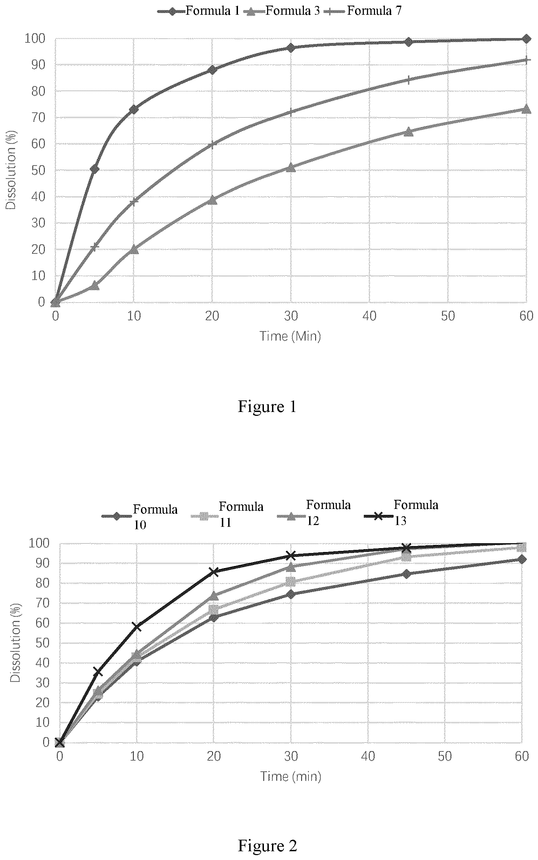

Conclusion: The dissolution profile of the nitroxoline oral solid tablet cores prepared from various formulas in Comparative Example 1 is as shown in Figure 1.

The results show that formula 1 and formula 2, which were firstly designed to investigate the dissolution of the bulk drug under natural release conditions, result in very fast dissolution rates. Formula 3, which was then designed to control the dissolution of the drug by delaying disintegration, result in very slow dissolution rate which is less than 80% in 60 minutes. Formula 4 to formula 7 were designed on the basis of formula 3. The results show that the tablet cores prepared with formula 7 has relatively moderate release rate.

### Example 1 Preparation of nitroxoline oral solid tablet cores

The above formula 7 with moderate release rate was taken as a sample. After a preliminary influencing factor test at 60°C for 10 days (the tablet cores were placed in a blast drying oven at 60°C for 10 days), it was found that the tablet could hardly disintegrate. Upon analysis and research, it is found that the reason may be that the nitroxoline as an ionic complexing agent deprives of the magnesium of magnesium stearate and frees the hydrophobic stearic acid with low melting point, which melts at the accelerated test temperature and thus blocks the water-permeable channels in the tablet, causing the reduction of the disintegration rate. In order to solve the problem of aging of the formula containing magnesium stearate, lubricants were screened and verified by using stearic acid as a positive control formula (formula 8) to obtain formula 8 to formula 10. At the same time, the amount of the disintegrating agent was also screened to obtain formula 11 to formula 13. Based on the total weight of the tablet core, the weight percentages of various components are shown in Table 2 below.

**Table 2 Weight percentage of various components in each formula**

| Functions | Materials/Formula | 8 | 9 | 10 | 11 | 12 | 13 |
|---|---|---|---|---|---|---|---|
| Active pharmaceutical ingredient | Nitroxoline (%) | 50 | 50 | 50 | 50 | 50 | 50 |
| Filler | Lactose (%) | 17.5 | 17.5 | 17.5 | 17.5 | 17.5 | 17.5 |
| Filler | Starch (%) | 28 | 27 | 27 | 26.5 | 26 | 25 |
| Disintegrating agent | Hydroxypropyl cellulose (%) | 2 | 2 | 2 | 2.5 | 3 | 4 |
| Binder | Starch (%) | 1.5 | 1.5 | 1.5 | 1.5 | 1.5 | 1.5 |
| Lubricant | Stearic acid | 1 | - | - | - | - | - |
| Lubricant | Sodium dodecyl sulfate (%) | - | - | 2 | 2 | 2 | 2 |
| Lubricant | Sodium stearyl fumarate (%) | - | 2 | - | - | - | - |

Preparation method for the nitroxoline oral solid tablet core:
(1) The nitroxoline, filler, disintegrating agent, binder and lubricant were respectively weighed according to the amount in the table above;
(2) The nitroxoline was sieved through a 10 mesh sieve and the other components were sieved through a 60 mesh sieve;
(3) Each amount of the binder was prepared into an aqueous solution at 5% mass concentration with purified water;
(4) Each amount of nitroxoline, filler and disintegrating agent were put into a wet granulation pan and mixed well, and then the aqueous solution of the binder obtained in step (3) was added. The mixture was stirred at high speed and discharged, which was then subjected to wet granulation by an oscillating granulator equipped with 20 mesh sieve. The granules were dried in a 60°C blast drying oven and then subjected to dry granulation by an oscillating granulator equipped with 20 mesh sieve to give an uniform granule;
(5) Each amount of the lubricant was added to the granules prepared in step (4) and mixed. The mixture was tableted by a rotary tablet press (equipped with 6 mm round punch) to give the nitroxoline oral solid tablet core.

### Example 2 Dissolution test of the nitroxoline oral solid tablet cores prepared in Example 1

According to Pharmacopoeia of the People's Republic of China, 2015 edition, Volume IV, General Requirements 0931, 1000 mL of 0.1 mol/L HCl was used as the dissolution medium, and the rotating speed of the automatic dissolution tester (model RC8MD, TDTF Company) was 50 revolutions per minute. The samples were collected at 5, 10, 20, 30, 45 and 60 minutes respectively, and the absorbance was measured at a wavelength of 369 nm by an UV spectrophotometer. The dissolution was calculated according to the external standard method.

Conclusion: The dissolution profile of the nitroxoline oral solid tablet cores prepared from various formulas in Example 1 is as shown in Figure 2.

The results show that the dissolution rates of the tablet cores prepared with different lubricant of formula 8 to formula 10 are basically the same. However, after a preliminary influencing factor test at 60°C for 10 days, it is found that the positive control tablet core of formula 8 can hardly disintegrate within 20 minutes, which supports the hypothesis that the nitroxoline causes the aging of the formula containing magnesium stearate. The release rates of Prescription 9 and Prescription 10 are almost unaffected. For formula 11 to formula 13, the dissolution rate is positively correlated with the amount of the disintegrating agent, and formula 11 is preferred.

### Example 3 Nitroxoline oral solid tablet cores and preparation thereof

Based on the total weight of the tablet core, the weight percentages of various components are shown in Table 3 below. The weight percentage of the binder is the weight percentage of the solid binder, and the binder used in the preparation is an aqueous solution of starch at 5 wt%.

**Table 3 Weight percentage of various components in each formula**

| Use | Component | Formula (weight %) | | | | | | | | | | | |
|---|---|---|---|---|---|---|---|---|---|---|---|---|---|
| | | A | B | c | D | E | F | G | H | I | J | K | L |
| Active ingredient | Nitroxoline | 30 | 30 | 35 | 40 | 45 | 50 | 55 | 40 | 50 | 60 | 65 | 65 |
| Filler | Lactose | 30 | 25 | 22 | - | 30 | - | 15 | 25 | 17.5 | 10 | 10 | 10 |
| | Starch | 35 | 35 | 30 | - | 15 | - | 25 | 30 | 26.5 | 23 | 20 | 15 |
| | Microcrystalline cellulose | - | - | | 50 | - | 40 | - | - | - | - | - | - |
| Disintegrating agent | Hydroxypropyl cellulose | 2 | 5 | 5 | 3 | 3 | 4 | 2 | 2 | 2.5 | 3 | 1 | 5 |
| Binder | Starch | - | 2 | 4 | 4 | 4 | - | - | 1 | 1.5 | 3 | - | 4 |
| | Hydroxypropyl methylcellulose | 2 | - | - | - | | 4 | 1 | - | - | - | 3.5 | |
| Lubricant | Sodium stearyl fumarate | - | 3 | 4 | 3 | - | 2 | 2 | - | - | - | - | 1 |
| | Sodium dodecyl sulfate | 1 | - | - | - | 3 | - | - | 2 | 2 | 1 | 0.5 | |

Preparation method:
(1) The nitroxoline, filler, disintegrating agent, binder and lubricant were respectively weighed according to the amount in the table above;
(2) The nitroxoline was sieved through a 10 mesh sieve and the other components were sieved through a 60 mesh sieve;
(3) Each amount of the binder was prepared into an aqueous solution at 5% mass concentration with purified water;
(4) Each amount of nitroxoline, filler and disintegrating agent were put into a wet granulation pan and mixed well, and then the aqueous solution of the binder prepared in step (3) was added. The mixture was stirred at high speed and discharged, which was then subjected to wet granulation by an oscillating granulator equipped with 20 mesh sieve. The granules were dried in a 60°C blast drying oven and then subjected to dry granulation by an oscillating granulator equipped with 20 mesh sieve to give an uniform granule;
(5) Each amount of the lubricant was added to the granules prepared in step (4) and mixed. The mixture was tableted by a rotary tablet press (equipped with 6 mm round punch) to give the nitroxoline oral solid tablet core.

### Example 4 Preparation of nitroxoline oral solid coated tablets

Nitroxoline has strong mobility and permeability. In order to investigate the isolating and masking performance of the film coating powder on the tablet core, the tablet core of formula I in Example 3 was selected for coating to screen the coating powder and the amount. The model of each coating powder and its weight gain percentage based on the total weight of the tablet core are shown in Table 4 below, wherein the gastric-soluble film coating premix is consisting of titanium dioxide, talc, polyethylene glycol 6000, hydroxypropyl methylcellulose and tartrazine aluminum lake.

**Table 4-1 Weight percentage of various components in formula I**

| Functions | Materials/Formula | I | | | | | |
|---|---|---|---|---|---|---|---|
| Active pharmaceutical ingredient | Nitroxoline (%) | 50 | 50 | 50 | 50 | 50 | 50 |
| Filler | Lactose (%) | 17.5 | 17.5 | 17.5 | 17.5 | 17.5 | 17.5 |
| Filler | Starch (%) | 26.5 | 26.5 | 26.5 | 26.5 | 26.5 | 26.5 |
| Disintegrating agent | Hydroxypropyl cellulose (%) | 2.5 | 2.5 | 2.5 | 2.5 | 2.5 | 2.5 |
| Binder | Starch (%) | 1.5 | 1.5 | 1.5 | 1.5 | 1.5 | 1.5 |
| Lubricant | Sodium dodecyl sulfate (%) | 2 | 2 | 2 | 2 | 2 | 2 |
| Coating agent | Gastric-soluble film coating premix (weight gain %) | 5 | 7 | 9 | 11 | 13 | 15 |

**Table 4-2 Weight percentage of various components in Formula I**

| Functions | Materials/Formula | I | | | | | |
|---|---|---|---|---|---|---|---|
| Active pharmaceutical ingredient | Nitroxoline (%) | 50 | 50 | 50 | 50 | 50 | 50 |
| Filler | Lactose (%) | 17.5 | 17.5 | 17.5 | 17.5 | 17.5 | 17.5 |
| Filler | Starch (%) | 26.5 | 26.5 | 26.5 | 26.5 | 26.5 | 26.5 |
| Disintegrating agent | Hydroxypropyl cellulose (%) | 2.5 | 2.5 | 2.5 | 2.5 | 2.5 | 2.5 |
| Binder | Starch (%) | 1.5 | 1.5 | 1.5 | 1.5 | 1.5 | 1.5 |
| Lubricant | Sodium dodecyl sulfate (%) | 2 | 2 | 2 | 2 | 2 | 2 |
| Coating agent | Polyvinyl alcohol (weight gain %) | 5 | 7 | 9 | 11 | 13 | 15 |

**Table 4-3 Weight percentage of various components in Formula I**

| Functions | Materials/Formula | I | | | | | |
|---|---|---|---|---|---|---|---|
| Active pharmaceutical ingredient | Nitroxoline (%) | 50 | 50 | 50 | 50 | 50 | 50 |
| Filler | Lactose (%) | 17.5 | 17.5 | 17.5 | 17.5 | 17.5 | 17.5 |
| Filler | Starch (%) | 26.5 | 26.5 | 26.5 | 26.5 | 26.5 | 26.5 |
| Disintegrating agent | Hydroxypropyl cellulose (%) | 2.5 | 2.5 | 2.5 | 2.5 | 2.5 | 2.5 |
| Binder | Starch (%) | 1.5 | 1.5 | 1.5 | 1.5 | 1.5 | 1.5 |
| Lubricant | Sodium dodecyl sulfate (%) | 2 | 2 | 2 | 2 | 2 | 2 |
| Coating agent | Opadry (weight gain %) | 5 | 7 | 9 | 11 | 13 | 15 |

Preparation method of the nitroxoline oral solid coated tablets:
(1) The nitroxoline, filler, disintegrating agent, binder, lubricant and coating agent were respectively weighed according to the amount of the table above;
(2) The nitroxoline was sieved through a 10 mesh sieve and the other components were sieved through a 60 mesh sieve;
(3) Each amount of the binder was prepared into an aqueous solution at 5% mass concentration with purified water;
(4) Each amount of nitroxoline, filler and disintegrating agent were put into a wet granulation pan and mixed well, and then the aqueous solution of the binder prepared in step (3) was added. The mixture was stirred at high speed and discharged, which was then subjected to wet granulation by an oscillating granulator equipped with 20 mesh sieve. The granules were dried in a 60°C blast drying oven and then subjected to dry granulation by an oscillating granulator equipped with 20 mesh sieve to give an uniform granule;
(5) Each amount of the lubricant was added to the granules prepared in step (4) and mixed. The mixture was tableted by a rotary tablet press (equipped with 6 mm round punch) to give the nitroxoline oral solid tablet core.
(6) The coating agent is formulated into a solid content of 12-16% with purified water. Coating of one layer is carried out in a coating pan at a speed of 3-15 rpm, an air inlet temperature of 55-75°C, a bed temperature at 30-50°C and a atomization pressure at 0.2-0.3 MPa, and the weight gain percentage by mass is as shown in Table 4-1, Table 4-2 and Table 4-3. The nitroxoline oral solid coated tablets were obtained.

### Example 5 Penetration test of nitroxoline oral solid coated tablets

Nitroxoline has strong mobility and permeability, and it easily dyes other materials in contact because it is yellow. In the present invention, the coated tablets with different weight gains prepared in Example 4 were respectively placed in white plastic bottle which was then placed in an oven at 60°C for 10 days for investigation. It was observed whether the nitroxoline would penetrate the coating film and dye the white plastic bottle yellow. The penetrability results are as shown in Table 5 below.

**Table 5 Penetration of nitroxoline to various coating agents**

| Model/weight gain | 5% | 7% | 9% | 11% | 13% | 15% |
|---|---|---|---|---|---|---|
| Gastric-soluble film coating premix | Penetrat ion in 10 days | No penetration in 10 days | No penetration in 10 days | No penetration in 10 days | No penetration in 10 days | No penetration in 10 days |
| Polyvinyl alcohol | Penetrat ion in 1 day | Penetration in 3 days | Penetration in 3 days | Penetration in 5 days | Penetration in 5 days | Penetration in 10 days |
| Opadry | Penetrat ion in 1 day | Penetration in 3 days | Penetration in 3 days | Penetration in 5 days | Penetration in 5 days | Penetration in 10 days |

It can be seen from Table 5 that the weight gain of polyvinyl alcohol and Opadry coating powder is 15%, and the coating can be penetrated at the high temperature of 60 °C for 10 days. Meanwhile, the gastric-soluble film coating premix has a weight gain of more than 7%, and the coating is not penetrated at the high temperature of 60°C for 10 days, showing that it has strong isolating and masking properties and can meet the needs.

### Example 6 Dissolution test of the nitroxoline oral solid tablets of the present invention

The tablets were prepared according to Formula H, I, and J in Example 3, and were coated with the gastric-soluble film coating premix according to the method of Example 4. The coating weight gain was 11%, and the batch was 10,000 tablets/batch.

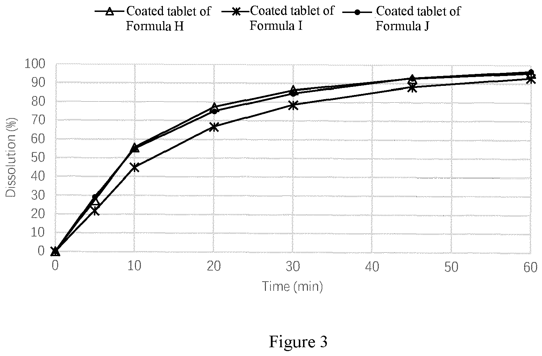

According to Pharmacopoeia of the People's Republic of China, 2015 edition, Volume IV, General Requirements 0931, 1000 mL of 0.1 mol/L HCl was used as the dissolution medium, and the rotating speed of the automatic dissolution tester (model RC8MD, TDTF Company) was 50 revolutions per minute. The samples were collected at 5, 10, 20, 30, 45 and 60 minutes respectively, and the absorbance was measured with an UV spectrophotometer. The dissolutions of coated tablets of formulas H, I and J were calculated according to the external standard method, and the dissolution profiles are as shown in Figure 3. The results show that the nitroxoline coated tablets of the three formulas H, I, and J have similar dissolution profiles, and the dissolution is almost completed at 60 minutes.

### Example 7 Quality inspection of the nitroxoline oral solid coated tablets of the present invention

The tablets were prepared according to formulas H, I, and J in Example 3, and were coated with the gastric-soluble film coating premix according to the method of Example 4. The coating weight gain was 11%, and the batch was 10,000 tablets/batch, wherein formula H of Example 3 was corresponding to the first batch, formula I of Example 3 was corresponding to the second batch, and formula J of Example 3 was corresponding to the third batch. The 3 batches were sampled for quality inspection.

Weight difference: 20 nitroxoline oral solid tablets were randomly taken and accurately weighed for the total weight to calculate the average tablet weight. Then each tablet was accurately weighed. The difference between the weight of each tablet and the average tablet weight should not exceed 7.5%.

Determination of the content: 10 nitroxoline oral solid tablets were randomly taken, accurately weighed and ground. An appropriate amount of the fine powder was accurately weighed and placed in a 100 mL measuring flask. Methanol was added to dissolve the powder and dilute to the mark. The solution was shaken well and filtered to prepare a solution containing 0.1 mg per 1 mL as the test solution. An appropriate amount of nitroxoline raw material was taken as control. Methanol was added to dissolve and dilute the nitroxoline to prepare a solution containing 0.1 mg per 1 mL, which was shaken well and used as the control solution. 10 µL of the test solution and the control solution were respectively injected into a liquid chromatograph and measured by using high-performance liquid chromatography (Pharmacopoeia of the People's Republic of China, 2015 edition, Volume IV, General Requirements 0512). The content was calculated with the peak area according to the external standard method. The content of nitroxoline should be 90.0-110.0%. The result is the average of samples in duplicate.

Determination of relative substance: 10 nitroxoline oral solid tablets were randomly taken and weighed for the average tablet weight. The tablets were ground into fine powder. The powder equivalent to 100 mg of the main drug (nitroxoline) was accurately weighed and put in a 100 mL measuring flask. Methanol was added to close to the mark. The mixture was ultrasonically extracted for 10 minutes and cooled to room temperature. The mixture was diluted to the mark with methanol, shaken well and centrifuged (3000 r/min). The supernatant was taken as the test solution. An appropriate amount of the test solution was diluted 100 folds with methanol as the control solution. 20 µL of the control solution and the test solution were respectively injected into the liquid chromatograph and measured by using high-performance liquid chromatography (Pharmacopoeia of the People's Republic of China, 2015 edition, Volume IV, General Requirements 0512). The sum of each impurity peak area should not exceed the main peak area of the control solution (1.0%). The result is the average of samples in duplicate. The weight difference, content and test results of the relative substance are shown in Table 6 below.

**Table 6 Results of quality inspection of the nitroxoline oral solid tablets of the present invention**

| Item/batch number | 1 | 2 | 3 |
|---|---|---|---|
| Appearance | Orange tablet | Orange tablet | Orange tablet |
| Weight difference (%) | ≤7.5 | ≤7.5 | ≤7.5 |
| Content (%) | 101.0 | 99.6 | 102.0 |
| Relative substances (%) | 0.03 | 0.03 | 0.03 |

Conclusion: It can be seen from Table 6 above that the appearance, weight difference, content and quality indicators of relative substances of the 3 batches of samples are basically consistent, and all meet the quality standard requirements.

Example 8 Accelerated stability test of nitroxoline oral solid tablets of the present invention

The tablets were prepared according to formula H, I, and J in Example 3, and were coated with the gastric-soluble film coating premix according to the method of Example 4. The coating weight gain was 11%, and the batch was 10,000 tablets/batch, wherein formula H of Example 3 was corresponding to the first batch, formula I of Example 3 was corresponding to the second batch, and formula J of Example 3 was corresponding to the third batch. The 3 batches of samples were put into oral solid high-density polyethylene bottles sealed with bottle caps with inductive aluminum cap lining, and investigated at a temperature of 40°C ± 2°C and a relative humidity of 75% ± 5% for 6 months.

Determination of content: the method was the same as the determination method in Example 7.

Determination of relative substance: the method was the same as the determination method in Example 7.

Determination of dissolution: 6 nitroxoline oral solid tablets were randomly taken. According to the dissolution and release test method (Pharmacopoeia of the People's Republic of China, 2015 edition, Volume IV, General Requirements 0931), 1000 mL of 0.1 mol/L hydrochloric acid was used as the dissolution medium, the rotation speed was 50 revolutions per minute, the operation was in accordance with the method, and samples were taken after 60 minutes. According to UV-visible spectrophotometry (Pharmacopoeia of the People's Republic of China, 2015 edition, Volume IV, General Requirements 0401), the absorbance was measured at a wavelength of 369 nm, the dissolution rate of each tablet was calculated, and the average value of the dissolution rates of 6 tablets was used as the result. The criterion is not less than 80% of the labeled amount in 60 minutes.

The test results of content, relative substances and dissolution are shown in Table 7 below.

**Table 7 Results of accelerated stability test of the nitroxoline oral solid tablets of the present invention**

| Batch number of sample | Time/Indicator | Appearance | Content (%) | Relative substances (%) | Dissolution (%) |
|---|---|---|---|---|---|
| 1 | 0 month | Orange tablet | 100.0 | 0.03 | 95.6 |
| | 1 month | Orange tablet | 98.7 | 0.03 | 96.1 |
| | 2 months | Orange tablet | 100.6 | 0.04 | 95.0 |
| | 3 months | Orange tablet | 98.9 | 0.04 | 95.7 |
| | 6 months | Orange tablet | 99.0 | 0.03 | 96.2 |
| 2 | 0 month | Orange tablet | 99.6 | 0.03 | 96.7 |
| | 1 month | Orange tablet | 100.7 | 0.03 | 97.4 |
| | 2 months | Orange tablet | 100.6 | 0.03 | 96.6 |
| | 3 months | Orange tablet | 98.5 | 0.03 | 95.6 |
| | 6 months | Orange tablet | 98.6 | 0.04 | 96.6 |
| 3 | 0 month | Orange tablet | 101.0 | 0.03 | 96.6 |
| | 1 month | Orange tablet | 99.6 | 0.03 | 95.3 |
| | 2 months | Orange tablet | 99.0 | 0.03 | 95.5 |
| | 3 months | Orange tablet | 99.4 | 0.03 | 95.8 |
| | 6 months | Orange tablet | 99.7 | 0.03 | 95.1 |

The results show that after the accelerated stability investigation for 6 months, the indicators of the 3 batches of samples have no significant changes compared with those at 0 month.

### Example 9 Long-term stability test of the nitroxoline oral solid tablets of the present invention

The tablets were prepared according to formula H, I, and J in Example 3, and were coated with the gastric-soluble film coating premix according to the method of Example 4. The coating weight gain was 11%, and the batch was 10,000 tablets/batch, wherein formula H of Example 3 was corresponding to the first batch, formula I of Example 3 was corresponding to the second batch, and formula J of Example 3 was corresponding to the third batch. The 3 batches of samples were put into oral solid high-density polyethylene bottles sealed with bottle caps with inductive aluminum cap lining, and investigated at a temperature of 25°C ± 2°C and a relative humidity of 60% ± 5% for 24 months.

Determination of content: the determination method was the same as that in Example 7.

Determination of relative substances: the determination method was the same as that in Example 7.

Determination of dissolution: the determination method was the same as that in Example 8.

The test results of content, relative substances and dissolution are shown in Table 8 below.

**Table 8 Results of long-term stability test of the nitroxoline oral solid tablets of the present invention**

| Batch number of sample (Formula) | Time/Indicator | Appearance | Content (%) | Relative substances (%) | Dissolution (%) |
|---|---|---|---|---|---|
| 1 (H) | 0 month | Orange tablet | 100.0 | 0.03 | 95.6 |
| | 3 months | Orange tablet | 99.3 | 0.03 | 94.3 |
| | 6 months | Orange tablet | 99.4 | 0.04 | 95.0 |
| | 9 months | Orange tablet | 99.6 | 0.03 | 94.3 |
| | 12 months | Orange tablet | 99.3 | 0.04 | 94.8 |
| | 18 months | Orange tablet | 99.5 | 0.04 | 94.2 |
| | 24 months | Orange tablet | 99.7 | 0.04 | 95.1 |
| 2 (I) | 0 month | Orange tablet | 99.6 | 0.03 | 96.7 |
| | 3 months | Orange tablet | 100.7 | 0.03 | 95.2 |
| | 6 months | Orange tablet | 99.1 | 0.04 | 96.3 |
| | 9 months | Orange tablet | 99.4 | 0.03 | 96.4 |
| | 12 months | Orange tablet | 99.2 | 0.03 | 96.1 |
| | 18 months | Orange tablet | 99.6 | 0.04 | 95.8 |
| | 24 months | Orange tablet | 99.4 | 0.03 | 95.2 |
| 3 (J) | 0 month | Orange tablet | 101.0 | 0.03 | 96.6 |
| | 3 months | Orange tablet | 99.5 | 0.03 | 95.0 |
| | 6 months | Orange tablet | 99.8 | 0.05 | 95.4 |
| | 9 months | Orange tablet | 99.8 | 0.03 | 95.6 |
| | 12 months | Orange tablet | 99.7 | 0.04 | 95.8 |
| | 18 months | Orange tablet | 99.4 | 0.04 | 94.8 |
| | 24 months | Orange tablet | 99.9 | 0.05 | 95.4 |

The results show that after the long-term stability investifation for 24 months, the indicators of the 3 batches of samples have no significant changes compared with those at 0 month.

## Claims

1. A pharmaceutical composition comprising nitroxoline, **characterized in that** the pharmaceutical composition comprises nitroxoline, a filler, a disintegrating agent, a binder, and a lubricant; wherein the lubricant is selected from one or two of sodium dodecyl sulfate and sodium stearyl fumarate.

2. The pharmaceutical composition comprising nitroxoline according to claim 1, **characterized in that**
the mass percentage of the nitroxoline in the pharmaceutical composition is 30%-65%, preferably 40%-60%;
and/or, the filler is one or more of lactose, starch, pregelatinized starch, microcrystalline cellulose, dextrin, mannitol, calcium hydrophosphate, mannitol and sorbitol; preferably one or more of lactose, starch and microcrystalline cellulose; more preferably lactose and/or starch; further more preferably lactose and starch, and the mass percentage of lactose in the pharmaceutical composition is 10%-25%, the mass percentage of starch in the pharmaceutical composition is 23%-30%; wherein, the lactose is preferably lactose monohydrate, and the starch is preferably corn starch;
and/or, the mass percentage of the filler in the pharmaceutical composition is 25%-65%, preferably 33%-55%;
and/or, the disintegrating agent is one or more of hydroxypropyl cellulose, cross-linked polyvinylpyrrolidone, sodium carboxymethyl starch and sodium croscarmellose, preferably hydroxypropyl cellulose, more preferably low-substituted hydroxypropyl cellulose;
and/or, the mass percentage of the disintegrating agent in the pharmaceutical composition is 1%-5%, preferably 2%-4%, more preferably 2%-3%;
and/or, the binder is one or more of starch, povidone, hydroxypropyl methylcellulose, hydroxypropyl cellulose and sodium carboxymethyl cellulose, preferably starch and/or hydroxypropyl methylcellulose, more preferably starch; wherein, the starch is preferably corn starch, and the hydroxypropyl methylcellulose is preferably E3 hydroxypropyl methylcellulose;
and/or, the mass percentage of the binder in the pharmaceutical composition is 1%-4%, preferably 1%-3%;
and/or, the lubricant is sodium dodecyl sulfate;
and/or, the mass percentage of the lubricant in the pharmaceutical composition is 0.5%-4%, preferably 1%-2%.

3. The pharmaceutical composition comprising nitroxoline according to claim 1 or 2, **characterized in that** the pharmaceutical composition comprises a coating agent which is selected from one or more of Opadry, polyvinyl alcohol, Eudragit and gastric-soluble film coating premix, preferably gastric-soluble film coating premix;
wherein, the gastric-soluble film coating premix preferably comprises titanium dioxide, talc, polyethylene glycol, hydroxypropyl methylcellulose and lake, more preferably a mixture consisting of titanium dioxide, talc, polyethylene glycol 6000, hydroxypropyl methylcellulose and tartrazine aluminum lake;
wherein, the weight gain of the coating agent is preferably 7%-15%, more preferably 10%-12%.

4. The pharmaceutical composition comprising nitroxoline according to any one of claims 1 to 3, **characterized in that**
the pharmaceutical composition comprises the following components in the following mass percentage: 30%-65% nitroxoline, 25%-65% filler, 1%-5% disintegrating agent, 1%-4% binder and 0.5%-4% lubricant;
preferably, the pharmaceutical composition comprises the following components in the following mass percentage: 40%-60% nitroxoline, 33%-55% filler, 2%-3% preferably 2%-2.5% disintegrating agent, 1%-3% binder and 1%-2% lubricant;
more preferably, in the pharmaceutical composition, the filler is lactose and starch, and the mass percentage of lactose in the pharmaceutical composition is 10%-25%, the mass percentage of starch in the pharmaceutical composition is 23%-30%; the disintegrating agent is hydroxypropyl cellulose, the binder is starch, and the lubricant is sodium dodecyl sulfate.

5. A nitroxoline oral solid tablet, **characterized in that** the tablet comprises nitroxoline as an active ingredient, a filler, a disintegrating agent, a binder and a lubricant; wherein the lubricant is selected from one or two of sodium dodecyl sulfate and sodium stearyl fumarate.

6. The nitroxoline oral solid tablet according to claim 5, **characterized in that** the tablet comprises 30-65% preferably 45-55% nitroxoline, 25-65% preferably 30-55% filler, 1-5% preferably 2-4% disintegrating agent, 1-4% preferably 1.5-3% binder and 0.5-4% preferably 1-3% lubricant by mass based on the total weight of the tablet.

7. A nitroxoline oral solid tablet, **characterized in that** the tablet comprises nitroxoline as an active ingredient, a filler, a disintegrating agent, a binder and a lubricant; the tablet is further coated with one or more layers of coating agent; wherein the lubricant is selected from one or two of sodium dodecyl sulfate and sodium stearyl fumarate.

8. The nitroxoline oral solid tablet according to claim 7, **characterized in that** the tablet comprises 30-65% preferably 45-55% nitroxoline, 25-65% preferably 30-55% filler, 1-5% preferably 2-4% disintegrating agent, 1-4% preferably 1.5-3% binder and 0.5-4% preferably 1-3% lubricant by mass based on the total weight of the tablet; and the percentage of weight gain of the coating agent is 7-15%, preferably 9-12% by weight.

9. The nitroxoline oral solid tablet according to any one of claims 5 to 8, **characterized in that** the filler is selected from one or more of lactose, starch, pregelatinized starch, microcrystalline cellulose, dextrin, mannitol, calcium hydrophosphate, mannitol and sorbitol, preferably lactose and/or starch; wherein, the lactose is preferably lactose monohydrate, and the starch is preferably corn starch.

10. The nitroxoline oral solid tablet according to claim 9, **characterized in that** the filler is lactose and starch, wherein the mass percentage of lactose is 10%-30%, preferably 15-25%, the mass percentage of starch is 15-35%, preferably 20-30% by mass based on the total weight of the tablet.

11. The nitroxoline oral solid tablet according to any one of claims 5 to 10, **characterized in that** the disintegrating agent is selected from one or more of hydroxypropyl cellulose, cross-linked polyvinylpyrrolidone, sodium carboxymethyl starch and sodium croscarmellose, preferably hydroxypropyl cellulose, more preferably low-substituted hydroxypropyl cellulose.

12. The nitroxoline oral solid tablet according to any one of claims 5 to 11, **characterized in that** the binder is selected from one or more of starch, povidone, hydroxypropyl methylcellulose, hydroxypropyl cellulose and sodium carboxymethyl cellulose, preferably starch and/or hydroxypropyl methylcellulose; wherein, the starch is preferably corn starch, and the hydroxypropyl methylcellulose is preferably E3 hydroxypropyl methylcellulose.

13. The nitroxoline oral solid tablet according to any one of claims 7 to 12, **characterized in that** the coating agent is selected from one or more of Opadry, polyvinyl alcohol, Eudragit and gastric-soluble film coating premix; preferably, the coating agent is gastric-soluble film coating premix; more preferably, the coating agent is gastric-soluble film coating premix, and the weight gain of the coating agent is 7%-15%, preferably 10%-12%.

14. The nitroxoline oral solid tablet according to claim 13, **characterized in that** the gastric-soluble film coating premix comprises titanium dioxide, talc, polyethylene glycol, hydroxypropyl methylcellulose and lake.

15. The nitroxoline oral solid tablet according to any one of claims 7 to 14, **characterized in that** the tablet comprises 30-65% preferably 45-55% nitroxoline as active ingredient, 10-30% preferably 15-25% lactose and/or 15-35% preferably 20-30% starch as filler, 1-5% preferably 2-4% hydroxypropyl cellulose as disintegrating agent, 1-4% preferably 1.5-3% starch or hydroxypropyl methylcellulose as binder, and 0.5-4% preferably 1-3% sodium dodecyl sulfate or sodium stearyl fumarate as lubricant by mass based on the total weight of the tablet; and the tablet is further coated with gastric-soluble film coating premix, the percentage of weight gain of which is 7-15%, preferably 9-12% by weight.

16. A preparation method of the pharmaceutical composition comprising nitroxoline according to any one of claims 1 to 4, **characterized in that** the preparation method includes the following steps:
S1 the nitroxoline, filler and disintegrating agent are mixed, which is then mixed with the solution containing the binder; the mixture is subject to wet granulation, drying and granulation to give the granules;
S2 the granulated granules are mixed with the lubricant and then subjected to tableting to give the tablets;
when the pharmaceutical composition contains a coating agent, coating may be performed after the tableting of step S2.

17. Use of the pharmaceutical composition according to any one of claims 1 to 4 or the nitroxoline oral solid tablet according to any one of claims 5 to 15 in the preparation of medicaments for the treatment of bladder cancer.
